# EUROPEAN PATENT SPECIFICATION

(11) **EP 2 419 117 B1**
(45) Date of publication and mention of the grant of the patent: **28.03.2018**
(21) Application number: 10764973.3
(22) Date of filing: 12.04.2010
(51) Int. Cl.: A61K 38/08, A61K 8/64, A61P 17/18, A61Q 19/08

(54) **Methionine sulfoxide peptide, compositions and methods of use**
METHIONINSULFOXIDPEPTID SOWIE ZUSAMMENSETZUNGEN DAMIT UND VERWENDUNGSVERFAHREN DAFÜR
Peptide de méthionine sulfoxyde, compositions et procédés d'utilisation

(30) Priority: 13.04.2009 US 168808 P
(43) Date of publication of application: 22.02.2012
(73) Proprietor: ELC Management LLC, New York, NY 10153 (US)
(72) Inventor: PELLE, Edward, Valley Stream New York 11580 (US); MAES, Daniel H., Huntington New York 11743 (US)
(74) Representative: Hirsch & Associés
(86) International application number: PCT/US2010/030749
(87) International publication number: WO 2010/120691

(56) References cited:
- EP-A1- 1 593 685
- WO-A2-93/25576
- WO-A2-2006/052775
- WO-A2-2010/082175
- WO-A2-2010/082177
- FR-A1- 2 338 925
- FR-A1- 2 391 191
- PELLE E ET AL: "Protection against UVB-induced Oxidative Stress in Human Skin Cells and Skin Models by Methionine Sulfoxide Reductase A", PROCEEDINGS OF THE II INTERNATIONAL CONFERENCE ON ENVIRONMENTAL STRESSORS IN BIOLOGY AND MEDICINE INTERNATIONAL CONGRESS ON PERSONALIZED MEDICINE 4TH ASIAN PACIFIC CONGRESS ON BRONCHOLOGY AND INTERVENTIONAL PULMONOLOGY MEDIMOND S R L, 2012, pages 19-24, XP009178146, & 2ND INTERNATIONAL CONFERENCE ON ENVIRONMENTAL STRESSOR IN BIOLOGY AND MEDICINE; SIENA, ITALY; OCTOBER 05 -07, 2011
- F. CABREIRO: "Methionine Sulfoxide Reductases: Relevance to Aging and Protection against Oxidative Stress", ANNALS OF THE NEW YORK ACADEMY OF SCIENCES, vol. 1067, no. 1, 1 May 2006 (2006-05-01), pages 37-44, XP055119989, ISSN: 0077-8923, DOI: 10.1196/annals.1354.006
- BROT N. ET AL.: 'Enzymatic reduction of protein-bound methionine sulfoxide' PROC. NATL. ACAD. SCI. vol. 78, no. 4, April 1981, pages 2155 - 2158, XP055093839
- MOZZICONACCI 0. ET AL.: 'Superoxide radical anions protect enkephalin from oxidation if the amine group is blocked.' FREE RADIC. BIOL. MED. vol. 43, no. 2, 10 April 2007, pages 229 - 240, XP022132570
- OGAWA F. ET AL.: 'The repair enzyme peptide methionine-S-sulfoxide reductase is expressed in human epidermis and upregulated by UVA radiation.' J. INVEST. DERMATOL. vol. 126, no. 5, May 2006, pages 1128 - 1134, XP055093840
- BALOG T. ET AL.: 'Met-enkephalin modulates resistance to oxidative stress in mouse brain.' NEUROPEPTIDES vol. 38, no. 5, October 2004, pages 298 - 303, XP004584054
- SCHALLREUTER K.U. ET AL.: 'Functioning methionine-S-sulfoxide reductases A and B are present in human skin.' J. INVEST. DERMATOL. vol. 126, no. 5, May 2006, pages 947 - 949, XP055093842

### SEQUENCE LISTING

<110> ELC Management LLC
<120> Methionine Sulfoxide Peptide, Compositions And Methods Of Use
<130> > 08.57
<150> > US61/168,808
   <151> 13 April 2009
<160> > 1
<210> > 1
   <211> 5
   <212> > PRT
   <213> Artificial Sequence
<220> >
   <223> MsrA up-regulator
<220> >
   <221> misc_feature
   <222> (5)
   <223> Met is singly oxidized
<400> 1

Tyr Gly Gly Phe Met
1               5

## Description

### FIELD OF THE INVENTION

The invention is in the field of anti-aging skin care. Specifically, it pertains to the use of a peptide containing an oxidized methionine residue to ameliorate the effects of oxidative stress in human skin.

### BACKGROUND

### Methionine

Methionine (C₅H₁₁NO₂S) is an electrically neutral, non-polar, hydrophobic α-amino acid with the chemical structure

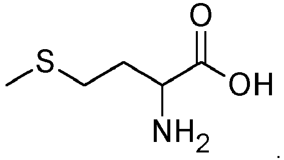

Methionine (Met) is proteinogenic. Of the proteinogenic amino acids, methionine has one of the lowest reported occurrences in proteins: 2.3%. Nevertheless, during protein translation, methionine is found at the N-terminal position of all eukaryote proteins. Met residues, as we shall see, protect the structure and function of a protein. It is an essential amino acid, so it must be ingested by humans.

Document "BALOG T. ET AL.: Met-enkephalin modulates resistance to oxidative stress in mouse brain. NEUROPEPTIDES vol; 38, October 2004 describes the effect of opioide peptide Met-enkephalin (MENK) on resistance to oxidative stress in the brain of 4, 10 and 18 months old CBA. Document BROT N. ET AL.: 'Enzymatic reduction of protein-bound methionine sulfoxide' PROC. NATL. ACAD. SCI. vol. 78, no. 4, April 1981, discloses anenzyme that catalyzes the reduction of methionine sulfoxide present in oxidize [Met]enkephalin. Document MOZZICONACCIO. ET AL.: 'Superoxide radical anions protect enkephalin from oxidation if the amine group is blocked.' FREE RADIC. BIOL. MED. vol. 43, April 2007 discloses that the pentapeptide methionine-enkephalin (Met-enk) is a natural opiate that inhibits signals of pain. Document SCHALLREUTER K.U. ET AL.: 'Functioning methionine-S-sulfoxide reductases A and B are present in human skin.' J. INVEST. DERMATOL. vol. 126, May 2006 discloses that methionine residues in the structure of proteins and peptides are especially sensitive to oxidation by hydrogen peroxide (H2O2) yielding both the (R) and (S) diastereomers of methionine sulfoxide. Document F. CABREIRO: "Methionine Sulfoxide Reductases: Relevance to Aging and Protection against Oxidative Stress", ANNALS OF THE NEW YORK ACADEMY OF SCIENCES, vol. 1067, no. 1, 1 May 2006 describes the implication of the Msr system in protection against oxidative stress and aging. WO2006052775 discloses peptides having a molecular weight of about 1034 daltons containing 10 amino acid residues have antitumor activity upon administration to mammals. The peptide has two oxygenated methionine residues. WO9325576 discloses peptides which substantially correspond in sequence to fragments of platelet-derived growth factor ("PDGF"). These peptides are used as agents for treatment of vascular diseases. FR2391191 and FR2338925 disclose peptides containing Met(O) that exhibit analgesic activity. WO2010082177 and WO2010082175 disclose peptidic compounds, in particular of the KXK type, for the cosmetic and dermopharmaceutic fields, that are able to improve the general state of the skin and its appendages. EP1593685 discloses antioxidant peptides that result from the proteolytic digestion of soy protein. The peptides may be used as additives in pharmaceutical or dietary compositions to protect compounds from oxidative degradation.

### Oxidation of Met and Skin Aging

In proteins, the sulfur-containing side chains of methionine residues, are subject to oxidation by reactive oxygen species (ROS) in the environment of the protein. Oxidation of methionine by ROS results in methionine sulfoxide (MetO), which has the following structure:

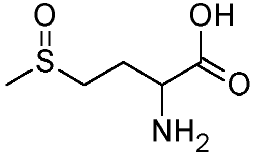

It is possible for the sulfur atom to be doubly oxidized, giving methionine sulfone (MetO₂). Methionine sulfone is a strong, biologically irreversible oxidant, and is distinguished from the milder methionine sulfoxide, which is biologically reducible. Throughout the specification, references to oxidized methionine refer to methionine sulfoxide, not methionine sulfone.

When a protein becomes oxidized at a methionine residue and this methionine is critical to the function and/or structure of the protein, then the protein's activity may become impaired. Oxidation of proteins has been linked to various disorders, including skin aging, where it has been identified as a major cause. See, for example, "The Repair Enzyme Peptide Methionine-S-Sulfoxide Reductase Is Expressed In Human Epidermis and Upregulated by UVA Radiation" (Ogawa et al. Journal of Investigative Dermatology (2006) 126, 1128-1134; published online 2 March 2006); which states: "the accumulation of oxidized proteins is considered a hallmark of cellular aging" and "The ability to reduce MetO appears to be essential for cells to survive in the presence of ROS". Furthermore, it is now known, that sun exposure is a significant contributor to ROS formation in human skin.

### Methionine Sulfoxide Reductase

The good news is that oxidation of a methionine amino acid residue is reversible, even in human skin, via mechanisms present within the cells. Oxidized peptides can be repaired. Reduction of methionine sulfoxide is catalyzed by methionine sulfoxide reductases (Msr), anti-oxidant enzymes that are found in most cells. With this understanding of the role of Msr, the theory has emerged that methionine residues exposed at protein surfaces, and Msr, form a redox cycle that regulates reactive oxygen species (ROS) in the cell. That is, protein Met residues are endogenous antioxidants, acting as scavengers of reactive oxygen species in cells, while Msr reduces the oxidized methionine back to a usable form. Thus, methionine oxidation at protein surfaces is not simply random damage, but part of a system to mitigate the effects of oxidative stress.

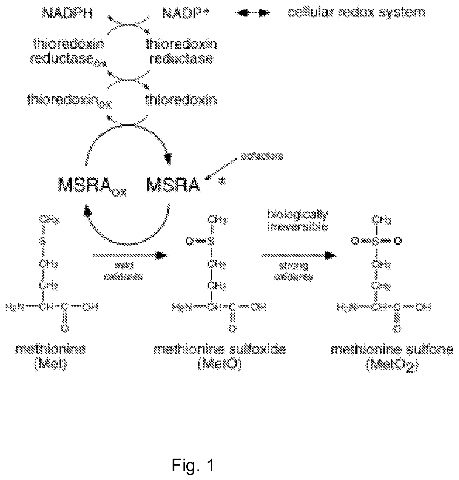

There are two forms of Msr, namely MsrA and MsrB. MsrA is involved in the reduction of the S-stereoisomer of Msr, while MsrB is involved in the reduction of the R-stereoisomer. Figure 1 is a schematic of the role of MsrA in reducing methionine sulfoxide back to methionine.

The presence of MsrA activity in epidermal keratinocytes was demonstrated by the applicant in 2003. Separately, the presence of MsrA activity in primary normal human epidermal keratinocytes and in HaCaT cells, and in human epidermis of test subjects, was reported by Ogawa *et al.* (reference above) in 2006. Both groups used RT-PCR (reverse transcription polymerase chain reaction) methods. Ogawa reported significant MsrA activity in primary human keratinocytes and HaCaT cells. Furthermore, Ogawa reported that MsrA is found preferentially in nucleated epidermal layers of human skin, rather than keratinized epidermal layers (i.e. stratum corneum) or dermal layers. Thus, within cells, including skin cells, Msr enzymes regulate protein function and prevent the accumulation of defective, mal-functioning proteins.

### Msr Down-Regulation Is Associated With Tissue Aging

Despite the Msr mechanism for repairing proteins, it has been shown that the amount of MetO in proteins increases with age. The implication is that the Msr system becomes compromised with age. As pointed out by Stadtman et al. (Methionine Oxidation and Aging; Biochimica et Biophysica Acta - Proteins & Proteomics; Volume 1703, Issue 2, 17 January 2005 (available online 9 September 2004), Pages 135-140):
"The change in levels of MetO may reflect alterations in any one or more of many different mechanisms, including (i) an increase in the rate of ROS generation; (ii) a decrease in the antioxidant capacity; (iii) a decrease in proteolytic activities that preferentially degrade oxidized proteins; or (iv) ***a decrease in the ability to convert MetO residues back to Met residues, due either to a direct loss of Msr enzyme levels*** or indirectly to a loss in the availability of the reducing equivalents (thioredoxin, thioredoxin reductase, NADPH generation) involved. The importance of Msr activity is highlighted by ***the fact that aging is associated with a loss of Msr activities in a number of animal tissues,*** and mutations in mice leading to a decrease in the Msr levels lead to a decrease in the maximum life span, whereas over-expression of Msr leads to a dramatic increase in the maximum life span."

Thus, aging leads to Msr down-regulation and Msr down-regulation leads to tissue aging. In recent years, many pathological disorders have been linked to loss Msr activity. These include, Alzheimer's and Parkinson's diseases, cataracts, vitiligo, α₁-antitrypsin related disorders (i.e. emphysema, skin aging) and decreased longevity.

### Msr Down-Regulation Is Associated With H₂O₂

Accumulation of hydrogen peroxide, H₂O₂ in the epidermal cells of vitiligo patients has been linked to reduced expression of the Msr enzymes. Conversely, when the increased levels of H₂O₂ in vitiligo patients were artificially reduced, Msr expression rebounded, approaching levels of healthy skin. Also, oxidative stress induced by H₂O₂ has been linked to reduced activity of Msr enzymes, and loss of ability to reduce MetO back to Met, in vitiligo patients. Furthermore, the loss of activity of MsrA and MsrB (but especially MsrA) in the presence of H₂O₂ (not necessarily in the skin of a vitiligo patient, just in the presence of H₂O₂), has also been demonstrated. (Schallreuter et al., "Methionine Sulfoxide Reductases A and B Are Deactivated by Hydrogen Peroxide (H2O2) in the Epidermis of Patients with Vitiligo"; Journal of Investigative Dermatology (2008) 128, 808-815; published online 18 October 2007).

Thus, reduced Msr levels and reduced Msr activity, are associated with elevated H₂O₂ levels. Increased oxidative stress is linked to reduced Msr levels.

### Msr Up-Regulation

Ogawa *et al.* have shown that exposure to UVA light significantly increases the expression of MsrA in the epidermis. This effect was also seen in cultured HaCaT cells. Response to UVA occurred in a time-, dose- and wavelength-dependent fashion. Likewise, Ogawa *et al.* report that up-regulation of MsrA occurred in cultured HaCaT cells, in response to low concentrations of hydrogen peroxide.

On the other hand, low doses of UVB were observed to down-regulate MsrA in HaCaT cells, within 72 hours after exposure, as reported by Ogawa *et al.* This is curious, because UVB exposure tends to induce production of hydrogen peroxide in the epidermis. Thus, one might have expected UVB and hydrogen peroxide to have similar effects. The picture is further muddled by the findings of Schallreuter *et al.,* discussed above, wherein hydrogen peroxide seems to down-regulate Msr in vitiligo patients. It is the applicant's opinion that the role of hydrogen peroxide in the epidermis and the effects of UVB exposure on the epidermis, vis-à-vis Msr, are not now fully understood.

### Sun Damaged Skin Cells

Throughout the specification, we may understand UVB to mean 280 - 320 nm wavelength and UVA to mean 320 - 450nm wavelength. Sunlight damages skin in either of two ways, termed direct damage and indirect damage. With direct damage, DNA in the skin directly absorbs photons from the UVB part of the spectrum. To a lesser extent, DNA also absorbs UVA photons. This absorption may cause mutations in the DNA sequence, which trigger a response within the skin cells. The response may include the formation of Sunburn Cells and the production of melanin. Only 8% of all melanoma occurrence is due to direct DNA mutation.

In contrast, indirect damage occurs when ultraviolet photons enter the skin and are absorbed by chromophores. In an excited state, chromophores enter into reactions that lead to the formation of reactive oxygen species. For example, in human skin, UVB exposure is associated with the production of hydrogen peroxide, while UVA is associated with production of singlet oxygen. If the skin is unable to maintain homeostasis by neutralizing the reactive species, then the reactive species will damage skin cell DNA and proteins through oxidation; for example, at the site of methionine. This damage to DNA and proteins is called oxidative stress and is a major cause of skin aging. Furthermore, 92% of all melanoma occurrence is due to indirect, oxidative DNA damage.

Unfortunately, it appears that certain sunscreens are able to penetrate the skin and contribute significantly to formation of reactive oxygen species. For example,
"The number of UV-induced (20 mJ cm⁻²) reactive oxygen species (ROS) generated in nucleated epidermis is dependent upon the length of time the UV filter octocrylene, octylmethoxycinnamate, or benzophenone-3 remains on the skin surface.... After each UV filter remains on the skin surface for *t* = 20 min, the number of ROS generated increases, although it remains below the number generated in the control. By *t* = 60 min, the filters generate ROS above the control. The data show that when all three of the UV filters penetrate into the nucleated layers, the level of ROS increases above that produced naturally by epidermal chromophores under UV illumination." (Hanson Kerry M.; Gratton Enrico; Bardeen Christopher J. (2006). "Sunscreen enhancement of UV-induced reactive oxygen species in the skin". Free Radical Biology and Medicine 41 (8): 1205-1212).

Thus, it has been suggested that use of certain sunscreens has contributed to an increased occurrence of melanoma and other oxidative damage. The present invention reduces the damaging effects of UV exposure without the risk of oxidative damage from some sunscreens. In fact, the present invention reverses oxidative damage of skin proteins.

### Skin Penetration

The stratum corneum represents the major penetration barrier for topically applied substances. The stratum corneum is a highly specialized tissue, made of approximately twenty layers of corneocytes embedded in a lipid matrix. The corneocytes are tightly packed and the space between them is filled by an organized structure of lamellar bilayers. The stratum corneum regulates the passage of lipophilic and hydrophilic substances through the skin. Many factors may determine whether a particular compound will be able to pass through the stratum corneum, and the rate at which it will pass through, to deeper layers of living epidermal cells. Some of these factors include size, polarity and solubility of the molecule, age and condition of the skin, the use of penetration enhancers and the route taken through the skin.

Regarding size, experience has shown that molecules have more and more difficulty penetrating the stratum corneum as the molecule's molecular weight increases beyond about 600 Daltons. Some sources place this cut off a bit lower, depending on the type and health of the skin. For example, one source suggests that "The largest molecules that can penetrate through intact skin have a molecular weight of 500." ("Routes for Skin Penetration", N. Dylan, Cosmetiscope Technical Notes 0504; accessed at http://www.nyscc.org/cosmetiscope/archive/tech0504.html on March 4, 2009). Therefore, in no way should 600 Daltons be taken as an absolute cut-off for skin penetration. Larger molecules may penetrate the skin. Nevertheless, increasing beyond about 600 Daltons, penetration through the stratum corneum by cosmetically acceptable means becomes increasingly problematic. Thus, if one was designing a carrier molecule to penetrate the skin, it would not be obvious to use a molecule having a weight of about 606 Daltons. Regarding polarity, in general, cationic species penetrate the skin better than anionic. Regarding solubility, lipophilic compounds tend to penetrate the stratum corneum faster then hydrophilic compounds.

The ability of topically applied compounds to enter and pass through the stratum corneum may be improved by known penetration enhancers, which reversibly decrease the skin's barrier resistance. Numerous chemical compounds have been evaluated for penetration enhancing activity, including sulphoxides (i.e. dimethylsulphoxide, DMSO), Azones (i.e. laurocapram), pyrrolidones (i.e. 2-pyrrolidone, 2P), alcohols and alkanols (i.e. ethanol or decanol), glycols (i.e. propylene glycol), surfactants and terpenes. These groups of penetration enhancers represent various modes of action and a variety of penetration channels through the skin barrier. Non-chemical means of enhancing barrier penetration include physical techniques (i.e. iontophoresis, sonophoresis), enzymatic techniques (altering certain aspects of stratum corneum lipids) and use of vesicular carriers (i.e. liposomes, niosomes etc.).

### Alpha 1-antitrypsin deficiency

Alpha 1-antitrypsin deficiency is a genetic disorder that leads to decreased α₁-antitrypsin (A1AT) activity in the blood and lungs. It is interesting to note that a study of the use of aerosolized A1AT, is underway. Aerosolized A1AT is inhaled into the lungs with the aim of reaching the lower respiratory tract. It is not yet known if the inhaled A1AT reaches the damaged elastin fibers in the lung. (see article "Alpha 1-antitrypsin deficiency", http://en.wikipedia.org/wiki/Alpha_1-antitrypsin_deficiency; last modified March 28, 2009.) This article does not disclose or suggest the use of a peptide having a C-terminal methionine sulfoxide residue, to upregulate Msr in the lungs. It does not disclose or suggest an aerosolized peptide having a C-terminal methionine sulfoxide residue.

Prior to the present invention, the applicant was unaware of any cosmetically acceptable topical composition that, when applied before exposure to the sun, protects the skin via Msr up-regulation. The applicant(s) knew of no prior art disclosing a cosmetically acceptable topical composition comprising a peptide having a terminal methionine sulfoxide, specifically a peptide that is about or smaller than about 600 molecular weight. The applicant(s) knew of no cosmetically acceptable method of reducing the damaging effects of UV light comprising the steps of applying to the skin a peptide that includes a terminal methionine sulfoxide.

### SUMMARY

A short polypeptide containing an oxidized methionine at its C-terminus is shown to inhibit the production of Sunburn Cells in living skin models and to upregulate methionine sulfoxide reductase. The short polypeptide is given by the following sequence: (SEQ ID NO: 1) Y - G - G - F - M - O Tyr - Gly - Gly - Phe - MetO. Cosmetically acceptable compositions and methods of using the polypeptide are disclosed. It is further conjectured that the oxidized peptide can repair oxidized alpha 1-antitrypsin, which, in turn, inhibits breakdown of connective tissue by elastase. This effect may have wide ranging ramifications, including, for example, repair of sun damaged skin and treatment of emphysema.

### DESCRIPTION OF THE FIGURES

Figure 1 is schematic of the role that MsrA plays in reducing methionine sulfoxide back to methionine.
Figure 2 shows the effect of Msr on UVB-induced hydrogen peroxide in normal human epidermal keratinocytes.
Figure 3 shows the effect of Msr on UVB-induced cell death, in normal human epidermal keratinocytes.
Figure 4 shows the positive effect of an oxidized peptide (MetO-enkephalin) on Msr upregulation, in normal human epidermal keratinocytes.
Figures 5 and 6 show the inhibitory effect of MetO-enkephalin on the production of Sunburn Cells caused by UVB radiation.
Figure 7 shows the low cytotoxicity of MetO-enkephalin in epidermal cells.
Figure 8 shows that α₁-antitrypsin inhibits elastase activity.
Figure 9 shows that oxidized α₁-antitrypsin cannot inhibit elastase activity.
Figure 10 shows that the elastase-inhibiting activity of α₁-antitrypsin, may be restored by MsrA, which reduces the oxidized α₁-antitrypsin.

### DETAILED DESCRIPTION

Throughout this specification, "comprise" and its conjugates shall be construed as open-ended, meaning that a group, list, collection or composition is not limited to the items explicitly named.

After establishing the presence of methionine sulfoxide reductase (Msr) in keratinocytes by conventional RT-PCR, experiments began with ways to upregulate this enzyme in a non-cytotoxic manner. The first material tried was oxidized methionine (the sulfoxide form), but results showed that the ratio of Msr to a housekeeping gene could not be raised. However, in these experiments, an oxidized peptide gave very good results.

The present invention is based, in part, on the following observations: 1. Msr inhibits UVB-induced hydrogen peroxide in NHEK and improves cell viability; 2. methionine sulfoxide (MetO), by itself, does not significantly upregulate Msr; 3. a small molecular weight polypeptide having a C-terminal methionine sulfoxide is able to upregulate Msr in normal human epidermal keratinocytes (NHEK); 4. the unoxidized version of this polypeptide does not significantly upregulate Msr; 5. in addition to upregulating Msr, the oxidized polypeptide is effective at inhibiting the formation of Sunburn Cells in UVB-irradiated living skin models; 6. by using the oxidized polypeptide, Msr is upregulated in a non-cytotoxic manner. Each of these will be discussed, in turn.

### 1. Msr inhibits endogenous and UVB-induced hydrogen peroxide in NHEK and improves cell viability

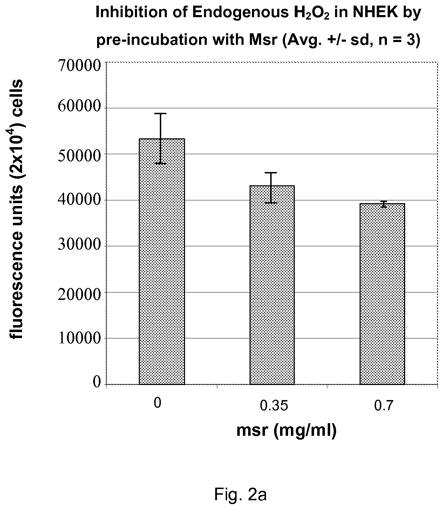

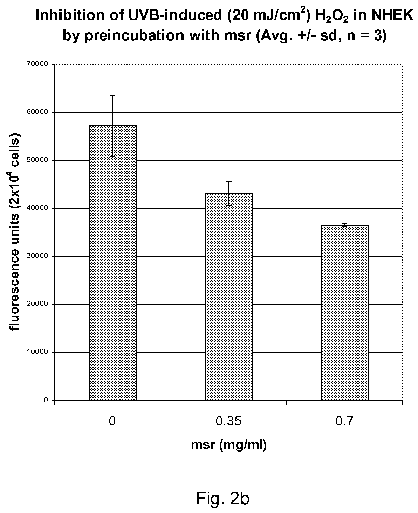

Figures 2a and 2b show the effects of Msr on endogenous and UVB-induced hydrogen peroxide, in normal human epidermal keratinocytes (NHEK). The NHEK were pre-incubated in Msr at the concentrations shown. After incubation, some cells were exposed to UVB (20mJ/cm²). As the chart shows, there was a significant reduction in levels of endogenous hydrogen peroxide and a significant reduction in the formation of hydrogen peroxide due to UVB exposure. Given that Msr inhibits UVB-induced H₂O₂, it was surmised that upregulating Msr, by the methods herein described, may be used prior to the onset of oxidative stress, as a preventive measure. Given that Msr reduces endogenous H₂O₂, it is established that upregulation of Msr, by the methods herein described, is anti-aging, with respect to keratinocytes. Thus, delivery of the oxidized peptide into the skin, seems to constitute a novel approach to anti-aging skin care.

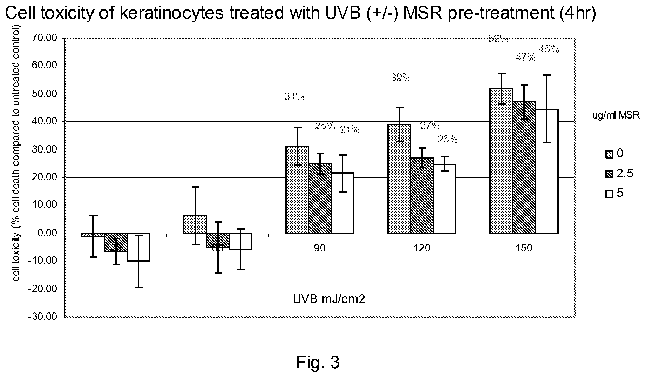

Figure 3 shows the effect of Msr on UVB-induced cell death in normal human epidermal keratinocytes. The NHEK were pre-incubated in Msr, for four hours, at the concentrations shown, followed by exposure to various levels of UVB. As the chart shows, the percent cell death as a result of UVB exposure is significantly decreased, compared to the untreated control. This result is observed at all tested levels of UVB exposure and Msr concentration.

### 2. Oxidized methionine (MetO), by itself, does not significantly upreaulate Msr in NHEK

On or about August of 2003, after establishing the presence of methionine sulfoxide reductase (Msr) in keratinocytes by conventional RT-PCR, experiments began with ways to upregulate this enzyme in a non-cytotoxic manner. The first material tried was oxidized methionine (the sulfoxide form). Results showed, however, that the level of Msr compared to a housekeeping gene, was not significantly raised. The applicants tried several times, but by February of 2004, it was clear that this oxidized amino acid would not induce Msr in keratinocytes. Oxidized bovine serum albumin, a comparatively large protein, was also tried, without success. However, in an experiment performed in February of 2004, an oxidized polypeptide gave very good results. Investigations continued along those lines.

### 3. Small molecular weight polypeptides having a C-terminal methionine sulfoxide are able to upregulate Msr in normal human epidermal keratinocytes (NHEK)

The oxidized peptide according to the present invention is given by the following sequence:
(SEQ ID NO. 1) Y - G - G - F - M-O Tyr - Gly - Gly - Phe - MetO
This polypeptide has molecular weight of approximately 606 and is oxidized at its C-terminal methionine residue.

The unoxidized version of this peptide is known as Met- enkephalin (CAS number: 58569-55-4; C₂₇H₃₅N₅O₇S).

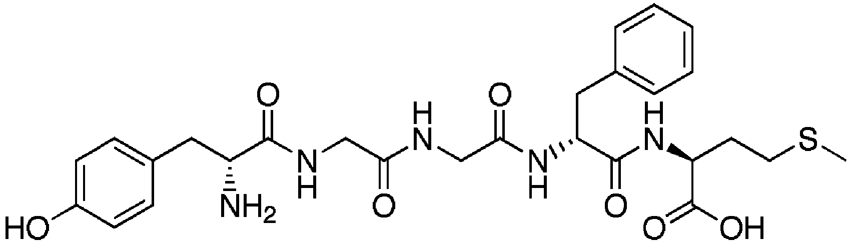

Met-enkephalin is a pentapeptide neurotransmitter, having a molar mass of 573.6611 g/mole. Met-enkephalin regulates pain and nociception in the body.

By "MetO-enkephalin" we mean the oxidized version of Met-enkephalin, oxidized at the sulfur of its C-terminal methionine residue, as shown in SEQ ID NO. 1.

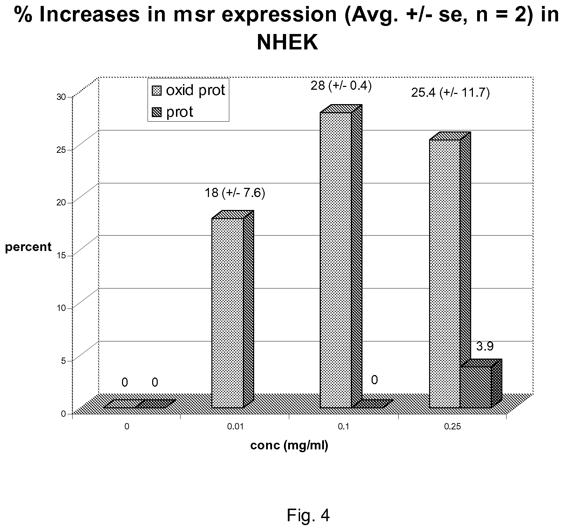

The following demonstrates that MetO-enkephalin upregulates Msr in normal human epidermal keratinocytes. NHEK cells were incubated overnight in culture medium (control) and in culture medium containing oxidized peptide and in culture medium containing unoxidized peptide (control). Concentrations (mg/mL) of the oxidized peptide included 0.01, 0.1 and 0.25. The unoxidized peptide (i.e. Met-enkephalin) was tested only at 0.25 mg/mL. After incubation, mRNA was extracted from the cells and cDNA was prepared by reverse transcription polymerase chain reaction (RT-PCR). Samples were probed for Msr via gel electrophoresis using β-microglobulin as a control. Results are shown in figure 4. Average increases in Msr expression were 18, 28 and 25.4 percent, for oxidized peptide concentrations of 0.01%, 0.1% and 0.25%, respectively. The result was confirmed with Western blotting, which showed increases of Msr mRNA in cells treated with the oxidized peptide.

### 4. The unoxidized polypeptide does not significantly upregulate Msr in NHEK

As may also be seen in figure 4, the unoxidized peptide caused no significant increase in Msr expression. Again, this result was confirmed with Western blotting, which showed no significant increases of Msr mRNA in cells treated with the unoxidized peptide.

### 5. The oxidized polypeptide is effective at inhibiting the formation of Sunburn Cells in UVB-irradiated living skin models

The effect of MetO-enkephalin oxidized peptide on UVB-irradiated living skin models was evaluated by examining the production of Sunburn Cells (SBC).

Methods: Epidermal Full Thickness 200 (EFT 200) living skin models were obtained from MatTek and incubated for 48 hours in media with or without 1 mg/ml of the MetO-enkephalin. The oxidized peptide can be stored at room temperature. Because the peptide is already oxidized, it has relative low reactivity in storage. Two ml of media were added beneath the living skin models, which were situated on a transwell membrane to allow the maintenance of an air/aqueous interface. After 48 hours, the living skin models were exposed to 100 mJ/cm² UVB, re-fed, and incubated overnight. The next day, the samples were fixed in 10% formalin and sent to Paragon Bioservices for histological sectioning and H&E staining. Five contiguous microscopic fields for each section were evaluated for the presence of SBC by counting the number of SBC and uninvolved cells and expressing SBC as a percentage of the total. Sunburn Cells are characterized by eosinophilic staining of the cytoplasm and pyknotic nuclei (condensed chromatin).

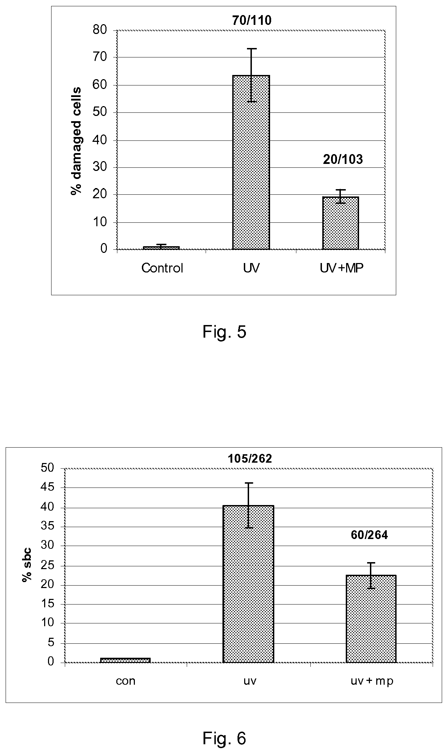

Results: The production of Sunburn Cells in the sample treated with 1mg/ml of MetO-enkephalin, was markedly reduced. The results of two separate trials of the same experiment are shown in figures 5 and 6, respectively. The average percent reduction in SBC from both trials was 31.1 %. Thus, a substantial amount of protection from the harmful effects of UVB radiation has been demonstrated by the MetO-enkephalin peptide.

The question was asked, is the observed reduction in Sunburn Cell formation due to absorbance of UVB by the peptide? The answer is certainly no. The UV spectrum of MetO-enkephalin is nearly identical to water. At 290 nm, there is an absorbance that translates into a k-value of 0.24 and an extinction coefficient of 145.5. However, this absorbance is slight, and therefore absorbance by the peptide did not significantly contribute to the reduction in SBC occurrence. Additionally, the peptide was applied beneath the living skin models and the exposures were carried out in 6-well plates having polystyrene lids, which effectively filter all UV radiation below approximately 300 nm. Thus, it may be concluded that the MetO-enkephalin peptide protects normal human epidermal keratinocytes from the harmful effects of UVB radiation, by other than absorbance of radiation. Without wishing to be bound by any one theory, it is thought that while UVB radiation increases the cellular concentration of MetO, the MetO-enkephalin is up-regulating Msr, which then reduces UVB-induced MetO back to Met.

### 6. By using the oxidized polypeptide, Msr is upregulated in a non-cytotoxic manner

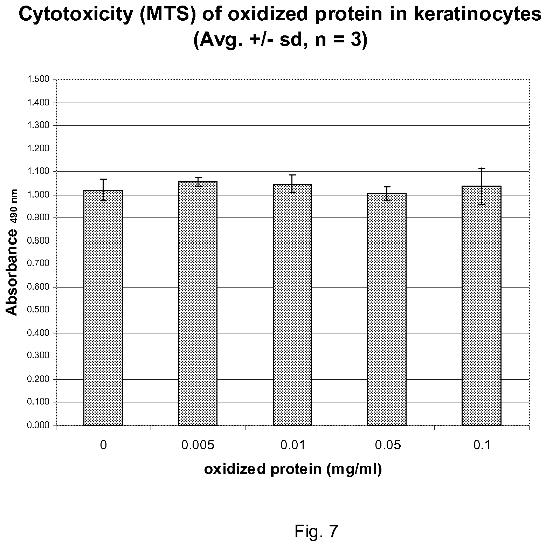

Figure 7 shows the cytotoxicity (or lack thereof) of MetO-enkephalin, at various concentrations, in keratinocytes. These results also demonstrate that the observed effects in keratinocytes following incubation with the oxidized peptide, were not due to cytotoxicity.

It was unexpected, and not inferred in the prior art, that a small, methionine-containing polypeptide, itself already oxidized at a C-terminal methionine residue, would have the observed effects. This is especially true, since, by itself, oxidized methionine is unable to upregulate Msr. In summary, the data suggests that MetO-enkephalin oxidized polypeptide upregulates Msr, protects living skin models against UVB stress by reducing SBC formation and inhibiting H₂O₂ production, and may reduce elastase-induced damage in the skin, as well as increase Msr levels in vitiligo patients.

### Repairing oxidized α₁-antitrypsin, inhibiting elastase with Msr

For the first time, Msr has been used to repair oxidized α₁-antitrypsin (a.k.a. A1AT; a.k.a. α1 proteinase inhibitor A1PI), with potentially wide ranging consequences. A1AT, a serin protease inhibitor made in the liver, inhibits a wide variety of proteases, not just trypsin. For example, A1AT protects tissues from the effects of enzymes released during inflammatory episodes. One such enzyme is elastase.

Elastase is a protease that breaks down elastin. Elastin is one of two main components of all connective tissue. Over time, if elastase is not checked, various connective tissue pathologies may result, depending on the location and function of the affected tissue. For example, excessive elastase activity may lead to saggy skin and elastosis; emphysema or chronic obstructive pulmonary disease in the lungs; and cirrhosis in the liver, just to name a few.

Under normal conditions, α₁-antitrypsin (A1AT) checks the activity of neutrophil elastase by maintaining elastase in an inactive form. A1AT does this by using its methionine 358 residue to bind elastase. But when A1AT becomes oxidized at its methionine 358 residue, the binding function of A1AT is impaired, and excessive breakdown of connective tissue ensues. Oxidation of the methionine 358 residue of A1At has been associated with increased levels of cellular hydrogen peroxide, inflammation, cigarette smoking and oxidative stress. What is needed, is a method of replenishing α₁-antitrypsin. Advantageously, it has been observed that Msr seems to repair oxidized α₁-antitrypsin and restore elastase inhibition.

Methods: An elastase assay from Molecular Probes was utilized and adapted for inhibition with α₁-antitrypsin. Fluorescence was measured by excitation at 485 nm and emission at 525 nm in a CytoFluor plate reader.

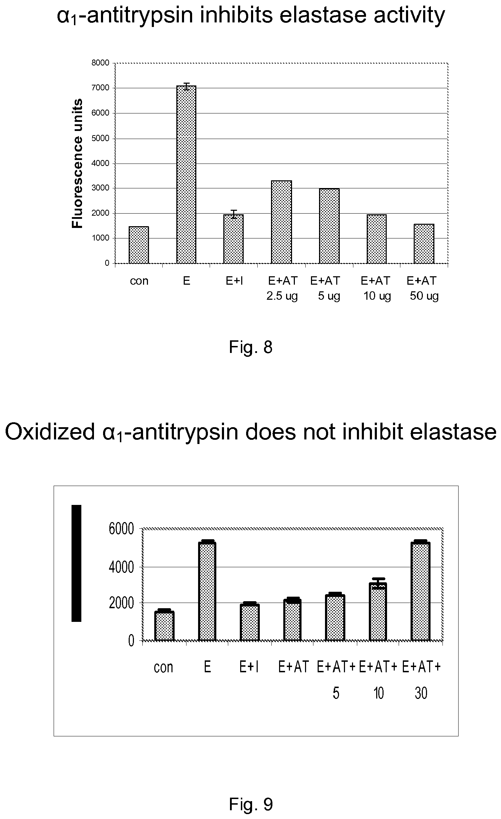

Results: Stage 1 of the experiment demonstrates that elastase activity is inhibited by α₁-antitrypsin. This was achieved by incubating 0.1 mg/ml elastin and 0.05 units/ml elastase with α₁-antitrypsin in a dose-dependent manner (2.5 - 50 ug/sample). The results are shown in Figure 8, where con = control; E = elastase; I = inhibitor (included with assay); AT = α₁-antitrypsin. Fluorescence decreases with increasing concentration of α₁-antitrypsin, indicating greater inhibition of elastase.

Stage 2 of the experiment demonstrates that hydrogen peroxide (H₂O₂) interferes with the ability of α₁-antitrypsin to inhibit elastase. This was done by oxidizing 10 µg of α₁-antitrypsin with hydrogen peroxide. Figure 9 shows that 5, 10, and 30 millimolar (mM) concentrations of H₂O₂, incubated for 3 hours at ambient temperature, inhibit α₁-antitrypsin in a dose dependent manner. At 30 mM of H₂O₂, almost all elastase inhibition provided by α₁-antitrypsin, is lost.

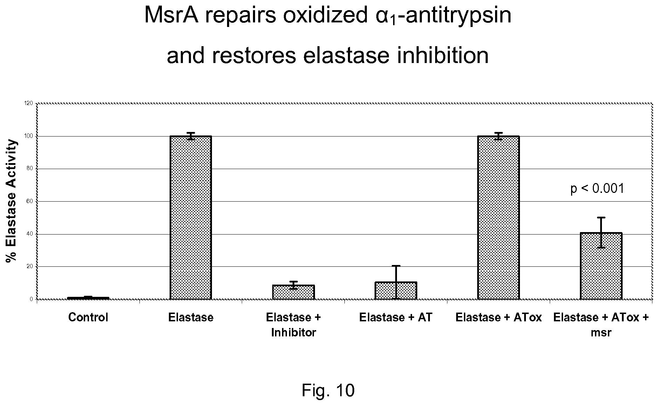

Stage 3 of the experiment demonstrates that the oxidation of α₁-antitrypsin can be reversed with msrA. This was achieved by incubating the A1AT that had been oxidized in 30 mM H₂O₂ with 0.7 mg/ml msrA and 10 mM DTT for 2 hours. The samples were then re-assayed as before in the elastase activity assay, along with the appropriate controls, such as, DTT alone, Msr alone, etc., and which had no effect. Figure 10 shows the result of this experiment. We start with 100% elastase. As before, α₁-antitrypsin (AT) removes about 90% of the elastase activity (bar labeled Elastase + AT). After oxidizing the α₁-antitrypsin, all of the elastase activity returns (bar labeled Elastase + ATox). Finally, the right-most bar in figure 10, shows that 0.7 mg/mL of MsrA led to the re-inhibition of over 59% of the elastase activity. Thus, MsrA was able to significantly restore elastase inhibition to oxidized α₁-antitrypsin with enzymatic precision.

Since Msr repairs oxidized α₁-antitrypsin, it is expected that the MetO-enkephalin oxidized peptide may be used to reduce elastase-induced damage to the skin, by upregulating epidermal Msr. Once MetO-enkephalin is delivered to and absorbed into the skin of a person in need of treatment, the MetO-enkephalin will upregulate Msr, which would then repair oxidized α₁-antitrypsin. The repaired α₁-antitrypsin would halt connective tissue breakdown by elastase.

Similarly, MetO-enkephalin may be expected to be useful in any condition or pathology that involves oxidation of the methionine 358 residue of α₁-antitrypsin. For example, it is expected that MetO-enkephalin may be useful in the treatment of emphysema. For example, MetO-enkephalin may be delivered to the lower respiratory tract in an aerosol vehicle, inhaled by a person in need of treatment for emphysema or chronic obstructive pulmonary disease. In the respiratory tract, MetO-enkephalin may upregulate Msr, which would then repair oxidized α₁-antitrypsin. The repaired α₁-antitrypsin would halt connective tissue breakdown by elastase.

It is further expected, that MetO-enkephalin may be useful in general, as an upregulator of Msr, in any tissues to which MetO-enkephalin can be delivered.

### Compositions

The benefits of the oxidized peptides herein described, are expected to be realized by applying to the skin, a composition comprising a cosmetically or pharmaceutically acceptable base and the oxidized peptides therein. The oxidized peptide is MetO-enkephalin. An "effective amount" of oxidized peptide(s) is an amount sufficient to upregulate Msr in quantities sufficient to prevent a defined level of oxidative tissue damage over a given surface area of skin. Effective amounts may be realized when the concentration of oxidized peptide(s) is as low as 0.01 % by weight of the preparation. Therefore, the average amount of topical preparation per square centimeter typically applied by a consumer could easily comprise an effective amount of oxidized peptide.

The MetO-enkephalin oxidized peptide may be incorporated into a wide range of cosmetically acceptable formulations suitable for topical application to the skin of a user. A first main restriction on the ingredients, form of composition and method of manufacture is that the final product must contain an "effective amount" of oxidized peptide, as described above. A second main restriction is that the final formulation should not reduce, to unacceptable levels, the oxidized peptide's ability to upregulate Msr or to inhibit the formation of Sunburn Cells.

In general, it will be possible, through ordinary skill in the art, to incorporate the oxidized peptide into aqueous and non-aqueous compositions. Aqueous compositions may be in the form of an emulsion, solution, suspension, dispersion, stick, gel or aerosol. If in the form of an emulsion, it may be a water-in-oil or oil-in-water emulsion. All aqueous forms of the composition may contain ranges of water, from least to most preferable, as follows: about: 1-99%, 5-99%, 1-90%, 10-99%, 5-90%, 1-85%, 5-85%, 10-90%, and 10-85%. When oil is present, there is 1-99%, preferably from about 5-90%, more preferably from about 5-75% of oil.

The composition may contain virtually any cosmetically acceptable ingredients. For example, it may contain sunscreens. Sunscreens include chemical UVA or UVB sunscreens or physical sunscreens in the particulate form. If present, the sunscreens may range from about 0.1 to 50%, preferably from about 0.5 to 40%, more preferably from about 1 to 35%. However, for a given level of protection, it may be possible to reduce the amount of sunscreen, because of the protection afforded by MetO-enkephalin, described above. Other optional ingredients include: humectants (i.e. glycols, sugars); surfactants (i.e. silicone-based or organic-based; if present, the surfactant may range from about 0.001 to 30%, preferably from about 0.005 to 25%, more preferably from about 0.1 to 20% by weight of the total composition); biological materials (i.e. %. fragments of cellular RNA or DNA, or probiotic microorganisms; if present such materials may range from about 0.001 to 30%, preferably from about 0.005 to 25%, more preferably from about 0.01 to 20); structuring agents; oils (i.e. volatile and non-volatile, silicone and non-silicone); vitamins; antioxidants; skin actives and more.

### Methods of Use

The oxidized peptide of the present invention may be used to repair damage due to oxidative stress or they may be used to prevent damage due to oxidative stress.

Repair - To repair skin damage due to oxidative stress, the oxidized peptides of the present invention may be applied to damaged skin. A composition according to the present invention, applied to the skin of a person in need of treatment for oxidatively damaged skin, will upregulate Msr, which will repair proteins in the skin that have become oxidized at their C-terminal methionine residues. The upregulated Msr may also repair α₁-antitrypsin (A1AT) that have become oxidized at their 358 methionine residues. Therefore, it is disclosed a method to reduce the amount of oxidized, methionine amino acid residues in human skin, whether these oxidized residues be in skin proteins, such as those found in keratinocytes, or in α₁-antitrypsin. The method includes the steps of applying an "effective amount" of MetO-enkephalin to skin that has oxidized methionine amino acid residues. The method may include the step of applying a composition comprising the oxidized peptide at a concentration of at least about 0.01%, by weight.

Prevention - To prevent skin damage due to oxidative stress, the oxidized peptide of the present invention may be applied to the skin prior to the onset of a specific oxidative stress. For example, if someone plans to spend a day at the beach, a composition according to the present invention, applied to the skin, will upregulate Msr and/or inhibit Sunburn Cell formation, which will reduce the damage that would have otherwise occurred due to UVA and/or UVB exposure. Therefore, it is disclosed a method of maintaining the amount of oxidized, methionine amino acid residues in human skin. The method includes the steps of applying an "effective amount" of MetO-enkephalin oxidized peptide to skin at risk of
experiencing the onset of oxidative stress; and, after the step of applying the oxidized peptide, exposing the skin to the risk. The method may include the step of applying a composition comprising the oxidized peptide at a concentration of at least about 0.01%, by weight.

Reducing Signs of Aging - A method of the invention measurably reduces one or more signs of skin aging. In this method, an effective amount of the MetO-enkephalin oxidized peptide is applied repeatedly to an area of skin manifesting one or more signs of skin aging. Repeated applications are made over days or weeks or months or years, at least until an improvement is observed in the skin. The oxidized peptide may be applied to the skin according to a defined schedule, i.e. one or more times per day for three weeks; or twice a day every other day, for six months. The applications may be carried out until a measurable reduction in one or more signs of aging is realized. Thereafter, applications may be stopped or altered to maintain the reduction in signs of aging. This method includes the step of regularly applying a composition comprising the oxidized peptide at a concentration of at least about 0.01%, by weight.

Signs of aging which may be measurably reduced include, but are not limited to all outwardly visible and tactile manifestations, as well as any other macro or micro effects due to skin aging. Such signs may be induced or caused by intrinsic factors or extrinsic factors, e.g., chronological aging and/or environmental damage. These signs may include, but are not limited to the following (to the extent that the following are caused by oxidative protein damage): textural discontinuities such as wrinkles; loss of skin elasticity (loss and/or inactivation of functional skin elastin), sagging (including puffiness in the eye area and jowls), loss of skin firmness, loss of skin tightness, loss of skin recoil from deformation, elastosis, collagen breakdown, and other histological changes in the stratum corneum, dermis, epidermis; discoloration (including under eye circles), blotching, shallowness, hyperpigmented skin regions such as age spots and freckles, keratoses, abnormal differentiation, and hyperkeratinization.

### Treating α₁-antitrypsin Disorders

It is disclosed a composition for use in methods of treatment of disorders due to oxidation of the 358 methionine residue on α₁-antitrypsin. In this method, an effective amount of MsrA or MetO-enkephalin is delivered to oxidized α₁-antitrypsin proteins. For example, the method may include applying MsrA or MetO-enkephalin to the skin of a person in need of such treatment, particularly in a vehicle that will allow the Msr or MetO-enkephalin to be absorbed into the skin. The disorder may be in the skin or deeper tissues. In the skin, the disorder may be elastosis or skin aging caused by breakdown of collagen.

A composition according to the present invention may be applied as part of a skin treatment regimen that includes other beneficial skin methods. For example, the skin is cleansed and treated with toner, after which the composition of the invention is applied. Also, the composition may be part of a kit comprising, for example, a cleanser, a toner, an exfoliant, and a composition of the invention.

## Claims

1. A composition comprising a cosmetically or pharmaceutically acceptable base and at least 0.01 % by weight of the oxidized peptide
(SEQ ID NO: 1) Y - G - G - F - M-O Tyr - Gly - Gly - Phe - MetO.

2. The composition of claim 1 wherein the composition is about 1 -99% of water.

3. The composition of claim 1 comprising a sunscreen.

4. A composition comprising a cosmetically or pharmaceutically acceptable base and at least 0.01 % by weight of the oxidized peptide
(SEQ ID NO: 1) Y - G - G - F - M-O Tyr - Gly - Gly - Phe - MetO
for reducing the amount of oxidized, methionine residues in human skin, by applying the composition to skin that has oxidized methionine residues.

5. The composition of claim 4 wherein the oxidized methionine residues are in keratinocytes.

6. The composition of claim 4 wherein the oxidized methionine residues are α₁-antitrypsin methionine 358 residues.

7. A composition comprising a cosmetically or pharmaceutically acceptable base and at least 0.01 % by weight of the oxidized peptide
(SEQ ID NO: 1) Y - G - G - F - M-O Tyr - Gly - Gly - Phe - MetO
for maintaining the amount of oxidized, methionine residues in human skin by
- applying the composition to skin at risk of experiencing the onset of oxidative stress; and, after the step of applying,
- exposing the skin to the risk.

8. The composition of claim 7 wherein the risk is exposure to UVA and/or UVB light.

9. A non-therapeutic method of reducing one or more signs of skin aging, comprising the steps of:
- providing a composition comprising a cosmetically or pharmaceutically acceptable base and at least 0.01 % by weight of the oxidized peptide
(SEQ ID NO: 1) Y - G - G - F - M-O Tyr - Gly - Gly - Phe - MetO;
- applying the composition, repeatedly, to an area of skin that manifests the one or more signs of skin aging, at least until an improvement in the skin is observed.

10. The method of claim 9 wherein the repeated applications are made over days or weeks or months or years, according to a defined schedule.

11. The method of claim 9 wherein the one or more signs of skin aging include wrinkles, loss of skin elasticity, loss of skin firmness, elastosis, collagen breakdown, and hyperpigmentation.

12. A composition comprising a cosmetically or pharmaceutically acceptable base and the oxidized peptide
(SEQ ID NO: 1) Y - G - G - F - M-O Tyr - Gly - Gly - Phe - MetO
or the oxidized peptide and MsrA
for treating α₁-antitrypsin disorders comprising the step of increasing MsrA levels in the tissues affected by the disorder by applying to the composition, in a vehicle that will allow the oxidized peptide to be absorbed into the skin.

13. The composition of claim 12, wherein the tissue disorder is vitiligo.

14. The composition of claim 12, wherein the tissue disorder is emphysema.

15. A composition comprising a cosmetically or pharmaceutically acceptable base and the oxidized peptide
(SEQ ID NO: 1) Y - G - G - F - M-O Tyr - Gly - Gly - Phe - MetO
for upregulating MsrA in human tissues by applying to the tissue the composition capable of penetrating the stratum corneum.

16. The composition of claim 15 wherein the upregulation occurs in epidermal keratinocytes.

## Patentansprüche

1. Eine Zusammensetzung, umfassend eine kosmetisch oder pharmazeutisch annehmbare Grundlage und mindestens 0,01 Gewichts-% des oxidierten Peptids
(SEQ ID NO: 1) Y - G - G - F - M-O Tyr - Gly - Gly - Phe - MetO.

2. Die Zusammensetzung nach Anspruch 1, wobei die Zusammensetzung von etwa 1 bis 99% aus Wasser besteht.

3. Die Zusammensetzung nach Anspruch 1, umfassend ein Sonnenschutzmittel.

4. Eine Zusammensetzung, umfassend eine kosmetisch oder pharmazeutisch annehmbare Grundlage und mindestens 0,01 Gewichts-% des oxidierten Peptids
(SEQ ID NO: 1) Y - G - G - F - M-O Tyr - Gly - Gly - Phe - MetO
um den Gehalt von oxidierten Methioninresten in der menschlichen Haut zu reduzieren, durch das Auftragen der Zusammensetzung auf eine Haut, die oxidierte Methioninreste aufweist.

5. Die Zusammensetzung nach Anspruch 4, wobei die oxidierten Methioninreste sich in Keratinozyten befinden.

6. Die Zusammensetzung nach Anspruch 4, wobei die oxidierten Methioninreste α₁-Antitrypsin-Methionin-358-Reste sind.

7. Eine Zusammensetzung, umfassend eine kosmetisch oder pharmazeutisch annehmbare Grundlage und mindestens 0,01 Gewichts-% des oxidierten Peptids
(SEQ ID NO: 1) Y - G - G - F - M-O Tyr - Gly - Gly - Phe - MetO
um den Gehalt von oxidierten Methioninresten in der menschlichen Haut beizubehalten durch:
- das Auftragen der Zusammensetzung auf eine, dem Risiko des Auftretens von oxidativem Stress ausgesetzte Haut; und, nach dem Schritt des Auftragens,
- das Aussetzen der Haut auf das Risiko.

8. Die Zusammensetzung nach Anspruch 7, wobei das Risiko die Aussetzung an UVA- und/oder UVB-Licht ist.

9. Ein nicht-therapeutisches Verfahren zur Reduzierung eines oder mehrerer Anzeichen von Hautalterung, welches folgende Schritte umfasst:
- Bereitstellen einer Zusammensetzung die eine kosmetisch oder pharmazeutisch annehmbare Grundlage und mindestens 0,01 Gewichts-% des oxidierten Peptids umfasst
(SEQ ID NO: 1) Y - G - G - F - M-O Tyr - Gly - Gly - Phe - MetO;
- wiederholtes Auftragen der Zusammensetzung auf einen Hautbereich der ein oder mehrere Anzeichen von Hautalterung aufweist, mindestens so lange bis eine Verbesserung festgestellt wird.

10. Das Verfahren nach Anspruch 9, wobei das wiederholte Auftragen, einem bestimmten Zeitplan gemäß, über Tage, Monate oder Jahre hin geschieht.

11. Das Verfahren nach Anspruch 9, wobei ein oder mehrere Anzeichen von Hautalterung Falten, Hautelastizitätsverlust, Hautfestigkeitsverlust, Elastose, Kollagen-Zusammenbruch und Hyperpigmentierung umfassen.

12. Eine Zusammensetzung, die Folgendes umfasst:
- eine kosmetisch oder pharmazeutisch annehmbare Grundlage und das oxidierte Peptid
(SEQ ID NO: 1) Y - G - G - F - M-O Tyr - Gly - Gly - Phe - MetO
- oder das oxidierte Peptid und MsrA
zur Behandlung von α₁-Antitrypsin-Störungen, umfassend die Schritte der Erhöhung der MsrA-Werte in den, von der Störung betroffenen Geweben, durch die Verwendung der Zusammensetzung in einem Träger der die Absorption des oxidierten Peptids durch die Haut erlaubt.

13. Die Zusammensetzung nach Anspruch 12, wobei die Gewebestörung Vitiligo ist.

14. Die Zusammensetzung nach Anspruch 12, wobei die Gewebestörung ein Hautemphysem ist.

15. Eine Zusammensetzung, umfassend eine kosmetisch oder pharmazeutisch annehmbare Grundlage und das oxidierte Peptid
(SEQ ID NO: 1) Y - G - G - F - M-O Tyr - Gly - Gly - Phe - MetO
zur positiven Regulierung von MsrA in menschlichen Geweben durch das Auftragen auf das Gewebe der Zusammensetzung, die in der Lage ist das Stratum corneum zu durchdringen.

16. Die Zusammensetzung nach Anspruch 15, wobei die positive Regulierung in epidermalen Keratinozyten erfolgt.

## Revendications

1. Composition comprenant une base cosmétiquement ou pharmaceutiquement acceptable et au moins 0,01 % en poids du peptide oxydé
(SEQ ID NO: 1) Y - G - G - F - M-O Tyr - Gly - Gly - Phe - MetO

2. Composition selon la revendication 1 où la composition est d'environ 1 à 99 % en eau.

3. Composition selon la revendication 1 comprenant un écran solaire.

4. Composition comprenant une base cosmétiquement ou pharmaceutiquement acceptable et au moins 0,01 % en poids du peptide oxydé
(SEQ ID NO: 1) Y - G - G - F - M-O Tyr - Gly - Gly - Phe - MetO
pour réduire la quantité de résidus de méthionine oxydée dans la peau humaine, par application de la composition sur la peau qui comporte des résidus de méthionine oxydée.

5. Composition selon la revendication 4, dans laquelle les résidus de méthionine oxydée sont dans des kératinocytes.

6. Composition selon la revendication 4, dans laquelle les résidus de méthionine oxydée sont les résidus de la méthionine 358 de l'α₁-antitrypsine.

7. Composition comprenant une base cosmétiquement ou pharmaceutiquement acceptable et au moins 0,01 % en poids du peptide oxydé
(SEQ ID NO: 1) Y - G - G - F - M-O Tyr - Gly - Gly - Phe - MetO
pour maintenir la quantité de résidus de méthionine oxydée dans la peau humaine par
- application de la composition sur la peau au risque de rencontrer l'apparition d'un stress oxydatif ; et, après l'étape d'application,
- exposition de la peau au risque.

8. Composition selon la revendication 7, dans laquelle le risque est l'exposition à une lumière à UVA et/ou UVB.

9. Procédé non thérapeutique de réduction d'un ou de plusieurs signes de vieillissement de la peau, comprenant les étapes de :
- fourniture d'une composition comprenant une base cosmétiquement ou pharmaceutiquement acceptable et au moins 0,01 % en poids du peptide oxydé
(SEQ ID NO: 1) Y - G - G - F - M-O Tyr - Gly - Gly - Phe - MetO ;
- application de la composition, de façon répétée, sur une zone de la peau qui manifeste un ou plusieurs signes de vieillissement de la peau, au moins jusqu'à l'observation d'une amélioration de la peau.

10. Procédé selon la revendication 9 dans lequel les applications répétées sont réalisées sur des jours ou des semaines ou des mois ou des années, selon un calendrier défini.

11. Procédé selon la revendication 9 dans lequel un ou plusieurs signes de vieillissement de la peau comprennent des rides, la perte de l'élasticité de la peau, la perte de la fermeté de la peau, une élastose, une dégradation du collagène, et une hyperpigmentation.

12. Composition comprenant une base cosmétiquement ou pharmaceutiquement acceptable et le peptide oxydé
(SEQ ID NO: 1) Y - G - G - F - M-O Tyr - Gly - Gly - Phe - MetO
ou le peptide oxydé et la MsrA
pour le traitement de troubles de l'α₁-antitrypsine comprenant l'étape d'augmentation des taux de MsrA dans les tissus touchés par le trouble par l'application de la composition, dans un véhicule qui permettra au peptide oxydé d'être absorbé dans la peau.

13. Composition selon la revendication 12, dans laquelle le trouble tissulaire est le vitiligo.

14. Composition selon la revendication 12, dans laquelle le trouble tissulaire est l'emphysème.

15. Composition comprenant une base cosmétiquement ou pharmaceutiquement acceptable et le peptide oxydé
(SEQ ID NO: 1) Y - G - G - F - M-O Tyr - Gly - Gly - Phe - MetO
pour la régulation positive de la MsrA dans des tissus humains par l'application au tissu de la composition capable de pénétrer dans la couche cornée.

16. Composition selon la revendication 15, dans laquelle la régulation positive survient dans les kératinocytes épidermiques.
